# EUROPEAN PATENT APPLICATION

(11) **EP 4 830 817 A1**
(43) Date of publication of application: **28.10.2026**
(21) Application number: 26182200.1
(22) Date of filing: 10.05.2023
(51) Int. Cl.: B01D 61/14, B01D 67/00, B01D 69/02, B01D 71/40, B01D 71/68

(54) **SURFACE TREATMENTS FOR MEMBRANES FOR PURIFICATION AND VIRUS FILTRATION OF MABS**

(30) Priority: 12.05.2022 US 202263341094 P
(62) Document number(s) of the earlier application(s) in accordance with Art. 76 EPC: 23726022.9 / 4 522 315
(71) Applicant: Merck Patent GmbH, 64293 Darmstadt (DE)
(72) Inventor: ELDO, Joby, Burlington, MA 01803 (US); CACACE, Benjamin, Burlington, MA 01803 (US); CARBRELLO, Christina, Burlington, MA 01803 (US)
(74) Representative: Haseltine Lake Kempner LLP

(57) **Abstract**

A surface treatment for a membrane, having a cross-linkable diacrylate solution used to make the cross-linked coating comprises a solution of at least one of 2.4-3.4% Hydroxypropyl acrylate and 1.1-2.1% polyethylene glycol diacrylate; 2.5-3.5% Bisphenol A ethoxylate diacrylate; 3.0-4.0% Bisphenol A ethoxylate diacrylate and 1.5-2.5% Hydroxypropyl acrylate; 1.5-2.5% Hydroxypropyl acrylate and 2.0-3.0% Polyethylene glycol diacrylate; 0.25-2.5% 2-Hydroxy-3-phenoxypropyl acrylate and 2.0-5.0% Polyethylene glycol diacrylate; or 3.0-4.0% Bisphenol A ethoxylate diacrylate. A filtration device for use with biological fluid, having a membrane; and a cross-linked coating on the membrane to form a surface modification or treatment, wherein the cross-linked coating is formed from a coating solution comprising a cross-linkable diacrylate.

## Description

### BACKGROUND

### Field of the Disclosure

Embodiments of the present disclosure relate to bioprocessing. More particularly, embodiments disclosed herein comprise the use of excipient and/or surface treatments on filtration membranes for enhancing flux.

### Description of Related Art

Biological fluids are processed in systems comprising containers, i.e., stainless steel containers and/or single-use containers or bioreactors, e.g., polymeric bags. These containers are sterile and used for the processing of biological products, whether in batch, semi-continuous, or continuous mode. The biological product(s) include cell cultures, empty capsids or viral vectors, plasmids, adeno-associated viruses and monoclonal antibodies (mAbs) among other products. These biological products must be purified using several processes, e.g., various chromatography, dead-end filtration and tangential flow filtration using porous membranes, for removing metabolic by-products and waste, viruses, and the like. Because the by-products, product aggregates, and wastes vary widely in size, fouling of the filters is a problem during bioprocessing.

Several manufacturers offer multiple filters retentive for viruses, for example, parvoviruses. The filters comprise membranes that are made from a variety of base polymers, e.g., poly (vinylidene fluoride), poly (ether sulfone), and some cellulosics. The surface of the membranes is often modified to be hydrophilic, and/or relatively low protein binding, etc. The surface modification may be a chemical coating process that, along with imparting hydrophilic properties, etc., may alter the pore size of the membrane. Typically, modifying the pore size of a membrane is undesirable and this can be a result of coating processes.

Some coating processes involve the formation of polymeric coatings comprising polymeric units from monomers and, cross-linking these polymeric units via one or more cross-linking approaches, such as chemical crosslinking agents in the presence of energy sources, such as gamma radiation, Ultraviolet, and/or E-beam. In some processes, the monomer itself can serve as the crosslinker. The polymer coatings used for this kind of surface modification are often based on acrylic and allylic type monomers and cross-linkers. The chemical and physical properties of the monomers/cross-linkers ultimately control the surface properties of the final filter.

There are known instances of process streams of biological fluids that cause lower initial flux and/or ongoing flux drop, i.e., fouling mechanisms of virus filters during the filtration of protein solutions. The adsorption of a protein within a biological fluid, onto the surface of the membrane, as a causative mechanism of fouling is a potential challenge during bioprocessing.

Some past prior art attempts of virus filtration processes include the use of excipients. For example, at least one method comprised a step for stabilizing downstream monoclonal antibody process intermediates by focusing on the prevention of large permanent aggregates that permanently blocked the filter pores. A theory has been postulated that in some instances concentration polarization occurs and accounts for flux behavior. The adsorption of aggregates to the surface of the pores as an alternate explanation was, however, dismissed as unlikely due to the hydrophilic coating of the membrane.

The use of excipients, particularly viscosity-reducing excipients, is generally known in final formulation processing where protein concentrations are higher and for multiple purposes of facilitating drug delivery and long-term shelf stability. Excipient molecules are known to suppress protein-protein interactions. However, the use of excipients has been limited. Many excipients do not provide significant filtration flux improvements despite being effective for other purposes. One potential reason for their ineffectiveness could be that conductivity is typically added to a biological fluid via charged molecular species, such as formulations that, e.g., contain sulfonic acid derivatives. This excess conductivity shields ionic interactions that could otherwise repel adsorptive interactions. These previous excipients do not significantly prevent the protein-protein and protein-membrane interactions that limit filtration flux.

While many monoclonal antibodies (mAbs) have minimal protein-protein and protein-membrane interactions that limit filtration flux under current bioprocessing concentrations, issues with filtration at low concentrations still exist. Moreover, advancements in other unit operations are pushing concentrations to higher levels where these interactions could become a limiting factor even more frequently.

Providing a membrane having a surface treatment chemistry that enhances use and performance, having significantly increased flux, is an advance in the art. Without intending to be bound by theory, it is thought that these advances are due to an unexpectedly high reduction of protein-membrane interactions. Additional advances of embodiments described in the disclosure also provide, without intending to be bound by theory, electrically neutral excipients that interact via pi-electrons and contribute little or no conductivity. Reducing protein-protein interactions lowers the effective size of the protein to be filtered, which, in some embodiments, in conjunction with reductions in protein-membrane interactions reduces pore constriction to effect an increase in processing flux. These two advancements in the art each provide significant advantages. Furthermore, in conjunction, provide synergistic advantages, providing increased filter flux when filtering viruses from various biological processes and products.

### SUMMARY

Some embodiments of a filtration membrane comprising a novel surface treatment formed from a diacrylate solution, substantially as shown in and/or described in connection with at least one of the figures, as set forth more completely in the claims, are disclosed. Some embodiments of the disclosure comprise a filtration device for filtering biological fluids for use with an excipient, a polyethersulfone membrane; and a cross-linked coating on the membrane to form a surface modification, wherein the cross-linked coating is formed from a coating solution comprising a cross-linkable diacrylate, and wherein the excipient provides pi-electron interactions. Some embodiments of a filtration membrane comprising a novel surface treatment chemistry and the use of an excipient are disclosed. Some embodiments include methods of using various excipients that provide pi-electron interactions to increase filter flux. It is to be understood that the filtration membranes have a novel surface treatment chemistry and/or use of pi-electron interaction excipients; which can be used to filter solutions containing mAbs as well as other biological modalities. Furthermore, the inventors, without intending to be limited by theory, have unexpectedly discovered that the concentration and interactions between protein and virus filtration membranes are viewed as a significant factor in the fouling of membranes. Some embodiments of the disclosure include a surface treatment for membranes, wherein the surface treatment comprises a coating solution used to make a cross-linked coating, further comprising at least one of 1) 2.4-3.4% Hydroxypropyl acrylate and 1.1-2.1% polyethylene glycol diacrylate; 2) 2.5-3.5% Bisphenol A ethoxylate diacrylate; 3) 3.0-4.0% Bisphenol A ethoxylate diacrylate and 1.5-2.5% Hydroxypropyl acrylate; 4) 1.5-2.5% Hydroxypropyl acrylate and 2.0-3.0% Polyethylene glycol diacrylate; 5) 3.0-4.0% Bisphenol A ethoxylate diacrylate; and 6) 0.25-2.5% 2-Hydroxy-3-phenoxypropyl acrylate and 2.0-5.0% Polyethylene glycol diacrylate.

Various provisions, aspects, and novel and inventive features of the present disclosure, as well as details of exemplary embodiments thereof, will be more fully understood from the following description and drawings.

### BRIEF DESCRIPTION OF THE FIGURES

FIG. 1 depicts a graph plotting throughput in kilograms per squared-meter of a surface treated membrane v. time, according to embodiments of the disclosure;
FIG. 2 depicts a graph plotting filtration flux in Liters per squared-meter per hour (LMH) of a surface treated virus filter membrane v. throughput in kilograms per squared-meters, according to embodiments of the disclosure;
FIG. 3 depicts a graph plotting throughout (in liters per squared-meter) v. time for three virus filter membrane devices filtering mAb according to embodiments of the disclosure;
FIG. 4 depicts a graph plotting filtrate volume (mL) after ten minutes at 2000 Relative Centrifugal Force (RCF) v. excipient concentration in millimoles per liter (mM), according to embodiments of the disclosure;
FIG. 5 depicts a graph plotting filtration flux of a surface treated membrane in LMH for mAbs v. throughput in kilograms per squared-meters, according to embodiments of the disclosure;
FIG. 6 depicts a graph plotting throughput in kilograms per squared-meter of a surface treated virus filter membrane v. time for a solution comprising mAb with or without caffeine excipient at mAb concentrations of 10g/L and 20 g/L, according to embodiments of the disclosure;
FIG. 7 depicts a graph plotting throughput in kilograms per squared-meter of a surface treated virus filter membrane v. time for a solution comprising mAb and Pyridoxine excipient at 300mM for three differing virus filtration devices, according to embodiments of the disclosure;
FIG. 8 depicts a table showing model Immunoglobulin G (IgG) protein nonspecific adsorption levels for various surface treated virus filter membranes reported in micrograms relative to membrane effective filtration area (EFA) in squared-centimeters, according to embodiments of the disclosure;
FIG. 9 depicts a first graph plotting throughput performance relative to test pressure in psi with a model test stream, according to embodiments of the disclosure;
FIG. 10 depicts a second graph plotting filtration flux in LMH of surface treated virus filter membrane v. throughput in liters per squared-meter for a model stream, according to embodiments of the disclosure;
FIG. 11 depicts a third graph plotting filtration flux in LMH of surface treated virus filter membrane v. throughput in liters per squared-meter versus for a model stream, according to embodiments of the disclosure;
FIG. 12 depicts a fourth graph plotting filtration flux of surface treated virus filter membrane devices in LMH v. throughput in liters per squared-meter for a model stream, according to embodiments of the disclosure;
FIG. 13 depicts a fifth graph plotting filtration flux of surface treated virus filter membrane devices in LMH v. throughput in liters per squared-meter for a model stream, according to embodiments of the disclosure;
FIG. 14 depicts a sixth graph plotting filtration flux of surface treated virus filter membrane devices in LMH v. throughput in liters per squared-meter for a model stream, according to embodiments of the disclosure; and
FIG. 15 depicts a seventh graph plotting filtration flux of surface treated virus filter membrane devices in LMH v. throughput in liters per squared-meter for a model stream, according to embodiments of the disclosure.

### DETAILED DESCRIPTION OF SOME EMBODIMENTS

So the manner in which the features disclosed herein can be understood in detail, a more particular description of the embodiments of the disclosure, briefly summarized above, may be had by reference to the appended drawings. It is to be noted, however, that the appended drawings illustrate only typical embodiments of this disclosure and are therefore not to be considered limiting of its scope, for the embodiments described and shown may admit to other equally effective embodiments. It is also to be understood that elements and features of one embodiment may be found in other embodiments without further recitation and that identical reference numerals are sometimes used to indicate comparable elements that are common to the figures.

The term wt% is defined as weight percent, i.e., weight of solute/weight of solvent multiplied by 100.

The terms "bioreactor," "bag," and "container" are generally used interchangeably within this disclosure. A flexible bioreactor, bag, or container connotes a flexible vessel that can be folded, collapsed, and expanded and/or the like, capable of containing, for example, a biological fluid. A single use bioreactor, bag, or container, typically also flexible, is a vessel that is used once and discarded.

The terms, "coating," "surface treatment," and "surface modification" are used interchangeably throughout this disclosure.

In some embodiments, a filtration device can indicate a membrane capable of filtering a biological fluid. In some embodiments, a filtration device can indicate one or more membranes housed within a housing that has at least one input for a biological fluid and at least one output and, optionally, a vent. In some embodiments, the filtration device is a virus filtration device.

The term "sterile" is defined as a condition of being free from contaminants and, particularly within the bioprocessing industry, free from bacteria, germs, and other microorganisms.

The term "adjuvant" within this disclosure is defined as a substance that enhances a body's immune response to, for e.g., an antigen.

The term "upstream" is defined as the condition of being in a physical position before another component with respect to the direction of the flow of a fluid. For example, upstream may indicate a process that occurs before a cell culturing process in a bioreactor. In some embodiments, the term "upstream" indicates a process for cultivating cells within a bioreactor. The term "downstream" is defined as the condition of being in a physical position after another component or process with respect to the direction of the flow of a fluid within a chain of processes or unit operations. From upstream to downstream, a series of unit operations in bioprocessing could include cell-culturing, capture chromatography, viral inactivation, an intermediate column chromatography step, ultrafiltration/diafiltration, a second chromatography step, a virus filtration step, and a purification/concentration step2

By way of example, embodiments of the disclosure comprise virus filters, e.g., virus filters with similar membrane pore structure to VIRESOLVE^{®} PRO (VPro), virus filter devices manufactured by EMD Millipore Corporation, Burlington, MA, USA, having improved surface treatment chemistry (ISC) and the use of novel excipients in the feed solution for improved filtration flux. Separately, the improved surface treatment provides a significantly improved filtration flux. Also, the use of excipients having pi-electron or hydrophobic interactions significantly improve filtration flux. When used in conjunction, a virus filtration filter that uses the novel surface treatment and excipients provides a synergistic improvement in filtration flux. Also, embodiments of the conjunction provide economical and practical processing at commercial scales. And, the excipients that were useful to improve flux through some membranes comprised at least one aromatic group. It is thought that aromaticity promotes pi-electron or hydrophobic interactions, which could play a role in preventing the protein-protein and protein-membrane interactions. Furthermore, excipients that allowed for a minimal conductivity were useful excipients in terms of improving transmembrane flux.

With some process streams, the virus filtration flux with the protein solution is much lower than the buffer flux and/or is dependent on the protein concentration. Operating at typical protein concentration values, for example approximately 10g/L, can be impracticably slow. Processing time is limited by the overhead costs of operating a manufacturing facility as well as other time dependent manufacturing considerations. In this limited time, the cost incurred is proportional to the filter sizing required for processing a set amount of feed material. Additionally, there are practical limitations such as the volumes of associated buffers for preparation to run and to recover product that also scale with filter installation membrane area. It is also to be understood that filters, comprising the membranes and/or when using excipients described herein, are also useful for continuous processes having longer run times but could still benefit from higher process flux and reduced filter footprint.

In some embodiments, the membrane comprises a polyethersulfone (PES) membrane. In some embodiments, the PES membrane is a porous membrane. In some embodiments, the porous membrane comprises a symmetric membrane or an asymmetric membrane. Embodiments disclosed herein provide novel compositions for surface treatments for removing microorganisms, especially viruses, from a biological fluid, and, in some embodiments, a purified monoclonal antibody solution. In some embodiments, the biological fluid is a chemically defined medium used for culturing cells expressing a protein of interest. In some embodiments, the biological fluid is a fractionated plasma product.

The compositions described herein are based on surface modifications of membranes resulting in membranes which exhibit reduced fouling by one or more components in a biological fluid having a certain modality, such as mAbs, fractionated plasma products, or a chemically defined cell culture medium, when such fluid or medium is filtered through the membrane.

In some embodiments, compositions described herein are directed to a porous membrane using an energy source. Exemplary energy sources include, but are not limited to, heat, electron beam, ultraviolet light and gamma radiation.

Also described herein are methods of using the described surface-treated membranes for removing a virus contaminant from monoclonal antibody solution, e.g., by filtering an antibody solution through the modified or surface-treated membrane.

Surface treatment of the membrane is achieved by the following steps. Initially, the chemistry mixes or protocols were prepared by mixing the monomer and the cross-linker at certain proportions in an appropriate solvent(s), e.g., water to form a coating solution. The range of concentrations generally used for monomer and cross-linker varied from 0-5 weight percentages. The chemistry mixture/coating solution was applied on the membrane surface by dipping the membrane in the corresponding chemistry mixture or otherwise applying or coating the membrane. This membrane further exposed to ultraviolet (UV), E-beam, gamma radiation, heat, and the like, for the polymerization and cross-linking reactions, resulting in the cross-linked coating of a chemistry on the membrane.

Many potential formulations, using various ranges of reagents, for coatings, are possible. Examples of some embodiments follow. Also, the ratios expressed in the following are not to be considered limiting of the embodiments.

Possible ranges for a first protocol, from which ISC 1 is formulated, comprise: 2.4-3.4% Hydroxypropyl acrylate (HPA) and 1.1-2.1% Polyethylene glycol diacrylate (PEGDA). The ratio of HPA to PEGDA ranges from 2.2-1.6.

Possible ranges for a second protocol, from which ISC 2 is formulated, comprise: 2.5-3.5% Bisphenol A ethoxylate diacrylate.

Possible ranges for a third protocol, from which ISC 3 is formulated, comprise: 3.0-4.0% Bisphenol A ethoxylate diacrylate and 1.5-2.5% Hydroxypropyl acrylate (HPA). The ratio of Bisphenol A ethoxylate diacrylate to HPA ranges from 2.0-1.6.

Possible ranges for a fourth protocol, from which ISC 4 is formulated, comprise: 1.5-2.5% Hydroxypropyl acrylate (HPA) and 2.0-3.0% Polyethylene glycol diacrylate (PEGDA). The ratio of HPA to PEGDA ranges from 0.75-0.83.

Possible ranges for a fifth protocol, from which ISC 5 is formulated, comprise: 3.0-4.0% Bisphenol A ethoxylate diacrylate.

Possible ranges for a sixth protocol, from which ISC 6 is formulated, comprise: 0.25-2.5% 2-Hydroxy-3-phenoxypropyl acrylate (HPPA) and 2.0-5.0% Polyethylene glycol diacrylate (PEGDA). The ratio of HPPA to PEGDA ranges from 0.12-0.62.

Other ranges and combinations are described without numbering of protocols for abbreviated reference. For example, embodiments within the scope of the disclosure include possible ranges comprising: 0.25-2.5% (2-Hydroxy-3-phenoxypropyl acrylate and 2.0-5.0% Polyethylene glycol diacrylate; 0.2-3.0% Tetrahydrofurfuryl acrylate and 1.0-2.5% Polyethylene glycol diacrylate; 0.2-3.0% (5-Ethyl-1,3-dioxan-5-yl)methyl acrylate and 1.0-2.5% Polyethylene glycol diacrylate; or 0.25-2.5% Propoxylated tetrahydrofurfuryl acrylate and 2.0-3.0% Polyethylene glycol diacrylate.

As tested, ISC 1 - ISC 6 were formulated as follows:
ISC 1: 4.5 wt% total chemistry. 2.86 wt% Hydroxypropyl acrylate Cas# 25584-83-2 (HPA) and 1.64 wt% Polyethylene glycol diacrylate Cas#26570-48-9 with number average molecular weight of 600 grams per mole (PEGDA600) (Ratio of HPA/PEGDA600: 1.75).
ISC 2 was formulated as 3 wt% Bisphenol A ethoxylate diacrylate, Cas#64401-02-1.
ISC 3 was formulated as 3.5 wt% Bisphenol A ethoxylate diacrylate and 2.0 wt% HPA.
ISC 4 was similar to ISC 1, but with a different component ratio 2.0 wt% HPA and 2.5 wt% PEGDA600.
ISC 5 was similar to ISC 2, but with slightly more monomer, e.g., 3.5 wt% Bisphenol A ethoxylate diacrylate.
ISC 6 was formulated as 0.25-2.5 wt% Hydroxy-3-phenoxypropyl acrylate and 2.0-5.0 wt% Polyethylene glycol diacrylate. In some embodiments, 1.0 wt% HPPA and 3.75 wt% PEGDA was used. In some embodiments, 1.0 wt% HPPA and 2 wt% PEGDA was used. In yet other embodiments, 0.25 wt% HPPA and 2wt % PEGDA was used.

Monoclonal antibody test solutions were selected to demonstrate the performance improvements possible with the invention. These mAbs have several characteristics that make them especially challenging to filter with conventional virus filters. They have high hydrophobicity causing protein-protein interactions and or protein-membrane interactions. Dilution can mitigate both of these interactions, but in commercial scale manufacturing, increases in cost and equipment size would be required for bioprocessing. Furthermore, after virus filtration, the products would need to be re-concentrated for storage and eventually patient dosing. Depending on how extreme the interactions are for any given protein they may become noticeable at conventional concentrations or only when operating a purification process under intensified conditions where concentrations are elevated. Known approaches of mitigating these interactions in the field of high concentration formulation include modification of pH and conductivity of the buffering system to enhance ionic repulsion. While some benefit was demonstrated by this approach, further and unexpected improvements were made using embodiments of the apparatuses and methods described herein. Furthermore, dynamic light scattering (DLS) to measure the interaction parameter (*k_{D}*) as defined by the slope of a linear regression plotted through diffusion coefficient v. protein concentration is a method of measuring protein-protein interactions. That method has been found to be a useful technique for predicting success of protein-protein interaction reduction approaches in improving virus filtration throughput. This technique has the advantage of requiring less feed material than throughput runs and therefore can be used to screen more options. Even while preparing small scale samples by performing buffer exchange in centrifugal filter units, the flow rate of the protein solutions can be correlated to the eventual throughput performance in a virus filtration operation. This was found to provide an additional approach to screening performance improvements through manipulation of buffer and use of additives.

For routine screening of a large variety of improved surface chemistry iterations, a model stream was utilized. A model stream for routine chemistry screening was prepared from commercially obtained human Immunoglobulin G (IgG) purified from blood plasma and lyophilized. In addition to choosing the feed and preparing it in such a way as to minimize virus filter fouling by large aggregates, the processing pressure was reduced to allow more time for adsorptive fouling to contribute to overall fouling.

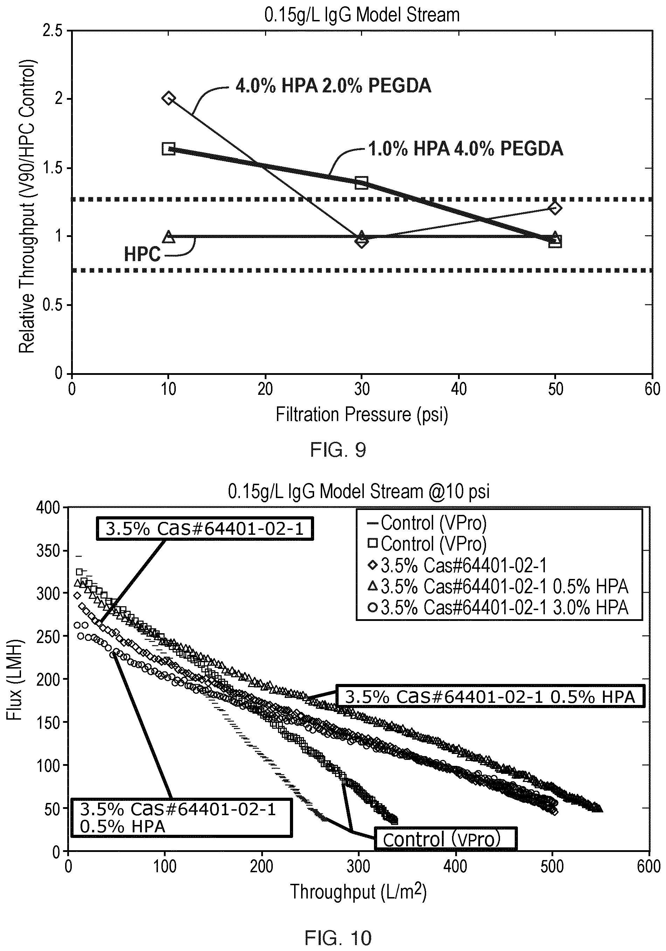

Thus, routine screening was performed on a single layer of the phobic base membrane modified with experimental chemistries. Volumetric throughput was measured versus time with constant 10 psi feed pressure, with end point being 90 % flux decay relative to buffer with no protein or 4 hours, whichever occurs first for any given sample. In Figure 9 it is shown how two chemistries can be differentiated from VPro control at 10 psi, but not as well at 30 psi, or 50 psi. With the model stream this specification was useful, although with other feed streams the impact of the surface chemistry can be seen at any typical operating pressure. Due to the potential for day-to-day variability in the test, results for each test are normalized to the throughput of the control device(s).

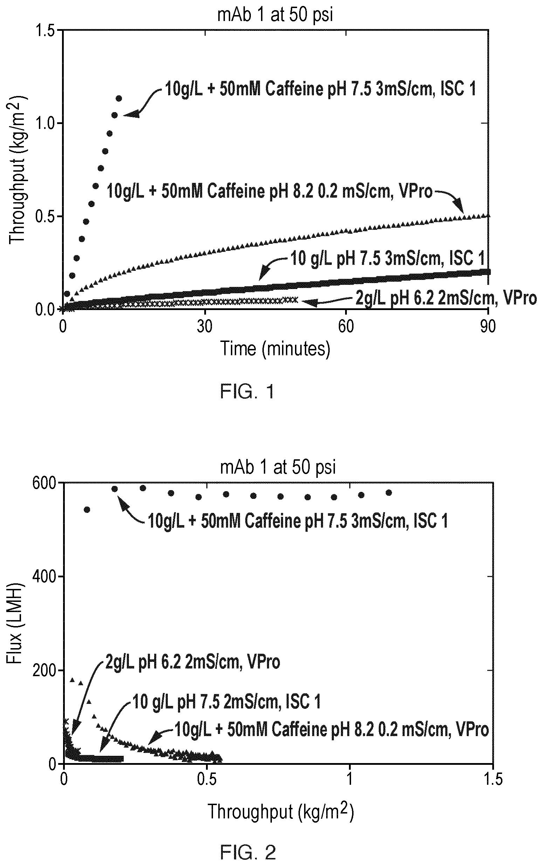

FIG. 1 depicts a graph plotting throughput in kilograms per squared-meter v. time of a surface treated membrane, according to embodiments of the disclosure. FIG. 1 demonstrates improvement in mass flux for mAb 1 using a Caffeine excipient and the ISC 1 device having a chemical surface treatment on the membrane allowing faster filtration and more throughput without fouling. The feed as is coming from the prior purification step has no measurable flow at 10g/L with VPro. By diluting without changing pH or conductivity a measurable flow rate is demonstrated with VPro. At 10g/L titrating to pH 7.5 allows improved flux with ISC 1. A full buffer exchange to much lower conductivity of 0.2 milliSiemens per centimeter (mS/cm) using a tris(hydroxymethyl)aminomethane (Tris) and Bicine buffer system at 20mM overall molarity up to pH 8.2 further enhances ionic repulsion allowing improvement in flux with VPro with addition of 50mM Caffeine. This improvement would require an extra tangential flow filtration unit operation to accomplish, thereby making it undesirable. Simple titration with Tris at a concentration of 2 moles per liter to increase to pH 7.5 would also otherwise make some improvement in ionic interactions while being an acceptable modification since it could be achieved without an additional unit operation. In stark contrast, when run with ISC 1 according to embodiments of the disclosure, a measurable flux is achieved at 10 g/L. When the titration is combined with the addition of 50mM Caffeine excipient and run on ISC 1 the flux is drastically increased, providing the best flux while maintaining concentration and avoiding additional unit operations.

FIG. 2 depicts data from FIG. 1 re-plotted as filtration flux in LMH v. throughput. By replotting in this way the advantage in volumetric flux can be seen for the ISC 1 when combined with use of an appropriate excipient.

The following conditions were applied for the trials shown in FIGS. 1-2. Prior to applying the feed solution to the virus filtration devices, processing through a multi-modal adsorptive membrane prefilter designed to remove large protein aggregates that would otherwise block the pores of the virus filter was conducted. After prefiltration, the fluid was run through the virus filters at constant pressure of 50 pounds per square inch (PSI).

VPro filtration devices, having the novel surface treatments, even absent the use of the novel excipients, are sufficient to demonstrate significantly superior performance with respect to filtration flux. While using either excipients or ISC 1 membrane alone demonstrates enhanced filtration flux, when employing both the surface treatment and an excipient, the resulting approximately 600 Liters per Meter squared of membrane per Hour (LMH) flux is 75% of the maximum possible value (buffer flux of 865 LMH at same 50 PSI). This is a 40X improvement over the approximate 15 LMH steady flux with either alone. The positive impact of the modified surface chemistry is an unexpected finding given the past published understanding regarding polarization, which is dependent only on the pore size and structure. Effective pore size can be reduced by addition of surface chemistry to a base structure. Notably, in this case no significant change was made to the pore size or structure. The similar pore size after having the novel surface treatment disposed thereon a similar starting membrane structure is confirmed by the similar buffer flux. While the excipients are known to reduce protein-protein interactions, the impact on protein-membrane interactions is not independently established, wherein making the synergistic improvement when combining surface treatments and excipient use even more unexpected.

It is to be further understood that many virus filters may incorporate the surface treatments described herein applied to the membrane used in their construction and/or use the novel excipients to modify feed streams and realize the synergistic effects thereof. For example, virus filter devices such as, VIRESOLVE^{®} PRO, Viresolve^{®} NFP and other membranes manufactured by the EMD Corporation, Burlington, MA, USA, along with other virus filters such as VIROSART HF and, PEGASUS PRIME.

While the examples shown here use Caffeine (which has aromaticity) as a novel excipient, it is believed that other aromatic excipient(s), which promote pi-electron interactions (without the addition of significant conductivity) show similar synergy, i.e., significantly enhanced filtration flux. At least two other suitable aromatic excipients are Pyridoxine and/or Phenylalanine and derivatives thereof. Additional aromatic excipients that could be effective include, for example, Pyridoxal, Pyridoxamine, Theobromine, Theophyline, Xanthine, Tyrosine, Tryptophan, Histidine, Histamine, Ascorbic acid, Folic acid, and Naphthalene-1-sulfonic acid and derivatives thereof. Concentrations of aromatic excipients used within a biological fluid are: 5mM - 300mM. Also, in some embodiments, combinations of aromatic excipients are used. For example, some embodiments comprise using pyridoxine and histidine together.

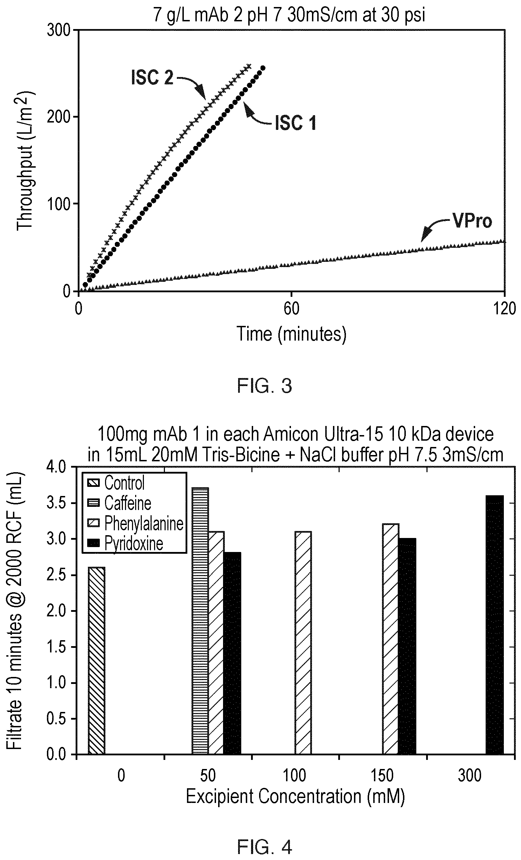

FIG. 3 depicts a graph plotting throughout (in liters per squared-meter) v. time of three virus filter devices filtering mAb 2 according to embodiments of the disclosure. Prior to applying the feed solution to the virus filtration devices, processing through a synthetic depth filter containing silica for the purpose of removing large protein aggregates that would otherwise block the pores of the virus filter was conducted. There are instances where improvements to the surface chemistry on the VPro base membrane alone are sufficient to get very good performance. In other words, improvements to a VPro device incorporating the novel surface treated membrane exhibits greater throughput. Furthermore, a VPro device having the novel surface treated membrane and using an aromatic excipient have even greater throughput. In this example, two different versions of the improved chemistry approach are presented - ISC 1; ISC 2. With no modification to the feed solution conditions from the prior purification step the ISC filters maintained higher flux and achieved higher throughput during the run time of 60 minutes when run at a constant pressure of 30 pounds per square inch (PSI). Two differing embodiments of the improved chemistry approach are presented. Two separate surface modifications, i.e., surface treatment chemistries, are carried out by different chemistry components. A first surface modification (ISC 1) was achieved by using a combination of an acrylic monomer (E.g., hydroxy propyl acrylate (HPA), e.g., Cas#25584-83-2) and an acrylic cross-linker (E.g., polyethylene glycol diacrylate (PEGDA), e.g., Cas#26570-48-9).

### Hydroxy propyl acrylate (HPA), e.g., Cas#25584-83-2

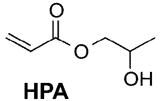

### Polyethylene glycol diacrylate (PEGDA), e.g., Cas#26570-48-9

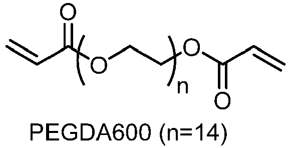

A second surface modification was performed with a single component which is acting as monomer as well as cross-linker (E.g., Bisphenol A ethoxylate diacrylate, e.g., Cas#64401-02-1).

### Bisphenol A ethoxylate diacrylate, e.g., Cas#64401-02-1

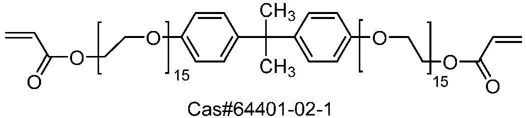

In both cases, a suitable chemistry formulation was identified from a series of studies performed at different formulation conditions. In both surface modifications, e-beam was used as the energy source to initiate the polymerization and cross-linking steps. Other energy sources as are known to those in the art are also contemplated herein.

FIG. 4 depicts a graph plotting filtrate volume after ten minutes at 2000 Relative Centrifugal Force (RCF) v. excipient concentration in millimoles per liter (mM), according to embodiments of the disclosure. After completing a buffer exchange into buffers containing different excipients at various concentrations, the amount of buffer passed through the membrane was measured at a fixed time point, according to embodiments of the disclosure. Even though in this lab method the mAb was retained by the membrane, the volume of buffer passed in a fixed time was correlated to virus filter flux performance where the mAb was passed through the virus filter. Higher buffer flux through the membrane in the centrifugal device predicted improved flux on the virus filter as would be used for the bioprocessing application. This method was used as a convenient screening method before running more complex flux runs with virus filters. FIG. 4 - as can be seen, shows results testing additional neutral cyclic excipients. Although at higher concentrations, e.g., 300mM, pyridoxine nearly reached the performance of caffeine at 50mM. Phenylalanine had a relatively lesser benefit of increased concentration, i.e., over a range of 50-150mM, while still outperforming the control. A base buffer of 20mM Tris-Bicine had sodium chloride (NaCl) added to reach a conductivity of 3mS/cm to match conditions that could be achieved via titration at scale as shown in FIG. 1 and FIG. 2.

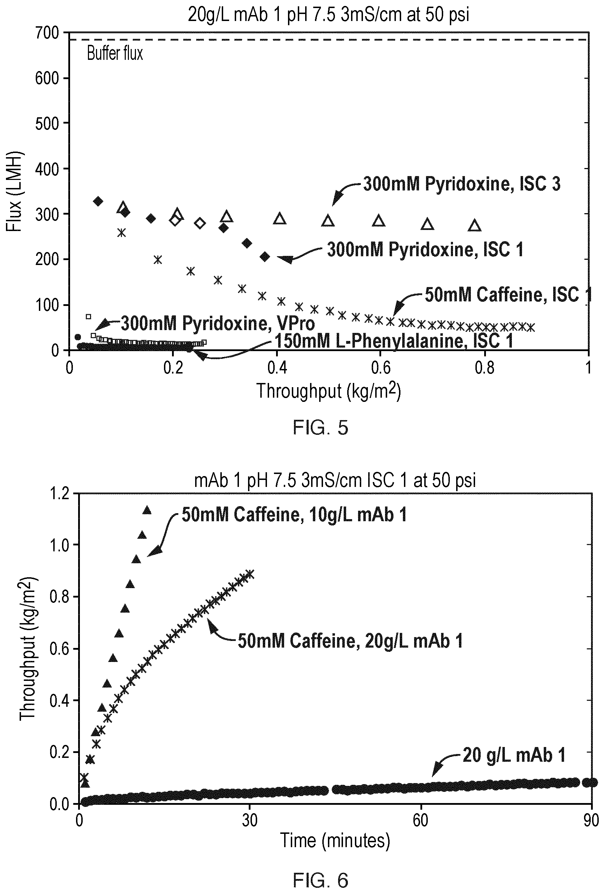

FIG. 5 depicts a graph plotting filtration flux of a surface treated membrane in LMH for mAbs v. throughput in kilograms per squared-meters, according to embodiments of the disclosure. As shown, L-Phenylalanine did not work as well as the other examples; 300mM Pyridoxine worked even better than Caffeine; ISC 3 sample variant was equivalent or better than ISC 1. One additional embodiment of the improved chemistry approach (ISC 3) is presented beyond those shown in other figures. This surface modification was achieved by using a combination of an acrylic monomer (E.g., hydroxy propyl acrylate (HPA), e.g., Cas#25584-83-2) and a component capable of acting as monomer as well as cross-linker (E.g., Bisphenol A ethoxylate diacrylate, e.g., Cas#64401-02-1). With the synergistic improvement between the excipient and improved surface chemistry the very difficult to filter mAb 1 could be processed at 300 LMH at the increased concentration of 20 g/L.

FIG. 6 depicts a graph plotting throughput in kilograms per squared-meter of a surface treated membrane v. time for a solution comprising mAbs, using Caffeine excipients with mAb 1 at 10g/L and 20 g/L, according to embodiments of the disclosure. The improved surface chemistry of ISC 1 allows measurable flow with mAb 1 at 20 g/L, but for a mAb with such a strong propensity to adsorb and at higher concentrations the flux is much improved when combining with an excipient such as Caffeine. Even this combination has reduced flux as can be seen by higher mass flux at the lower concentration of 10 g/L for mAb 1.

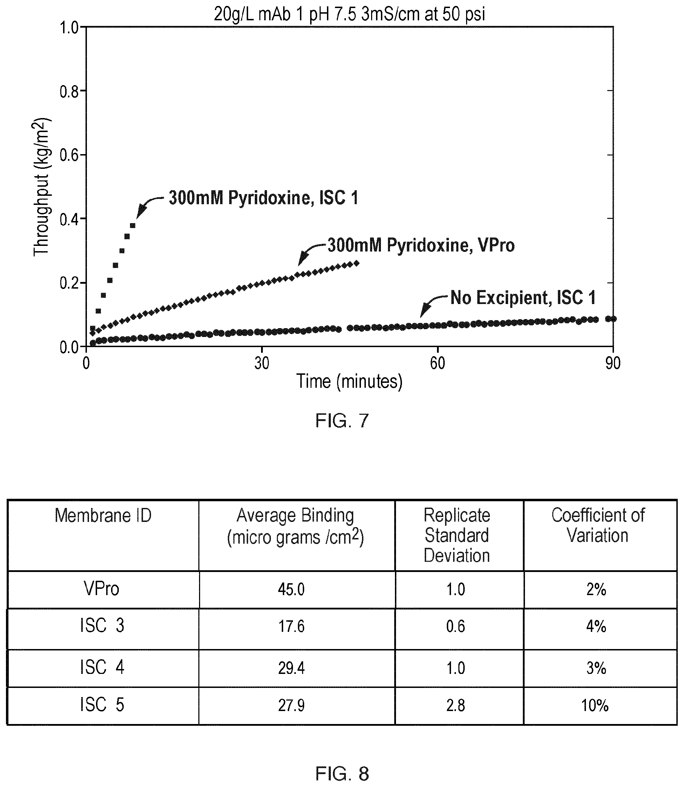

FIG. 7 depicts a graph plotting throughput in kilograms per squared-meter of a surface treated membrane v. time for a solution comprising mAb 1, using Pyridoxine excipients at 300mM for three differing virus filtration devices, according to embodiments of the disclosure. FIG. 7 shows a difficult to filter mAb 1 - combining excipient with improved surface chemistry virus filter.

ISC 1 by itself is not sufficient to run mAb 1 at 20 g/L. 300mM Pyridoxine by itself with standard VPro is better than ISC 1 by itself. Using both the Pyridoxine excipient and ISC 1 gives the best performance.

### Caffeine:

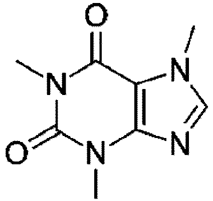

### L-Phenylalanine:

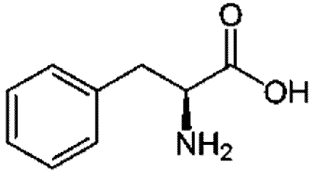

### Pyridoxine:

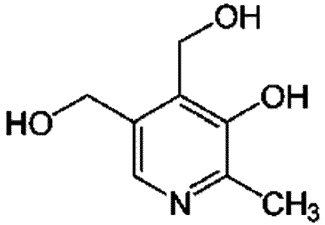

FIG. 8 depicts a table showing model Immunoglobulin G (IgG) protein nonspecific adsorption levels. Specifically, 1 g/L protein solution of Goat Gamma Globulin spiked with approximately 106 counts per minute (cpm) of 125I-Goat Anti Rabbit IgG labeled protein in a phosphate-buffered saline (PBS) pH 7.4 solution. Triplicate samples of membrane were measured with results reported relative to membrane effective filtration area (EFA). Reduction in adsorption of a relevant protein is confirmed by this analytical method showing reductions with ISC 3 having the lowest protein binding.

FIG. 9 depicts a graph plotting throughput performance relative to test pressure in psi with a model test stream, according to embodiments of the disclosure. The plotted data illustrates why subsequent testing with model stream is run at 10 psi where samples can be better differentiated. FIG. 9 depicts throughput of a test of filtration devices containing a single layer of surface modified membrane with test end point being defined as 90 % flux decay relative to buffer with no protein or 4 hours, whichever comes first for any given sample device. Due to the potential for day-to-day variability in the test, results for each test are normalized to the throughput of the control device or an average of multiple control devices. The model stream was prepared from commercially obtained human IgG purified from blood plasma and lyophilized. It is shown how two improved surface chemistries can be differentiated from VPro control at 10 psi, but not as well at 30 psi, or 50 psi according to embodiments of the disclosure. As shown, membranes prepared with Hydroxypropyl acrylate (HPA) and Polyethylene glycol diacrylate (PEGDA) achieved higher throughput than the VPro Control when tested at 10 psi.

FIG. 10 depicts a graph plotting filtration flux of a single layer of surface treated membrane in LMH v. throughput in liters per squared-meters for a model stream operated at 10 psi, according to embodiments of the disclosure. As shown, membranes prepared with a component capable of acting as monomer as well as cross-linker (E.g., Bisphenol A ethoxylate diacrylate, e.g., Cas#64401-02-1) with and without a range of concentrations of HPA (E.g., hydroxy propyl acrylate (HPA), e.g., Cas#25584-83-2) achieved higher throughput than the VPro Control.

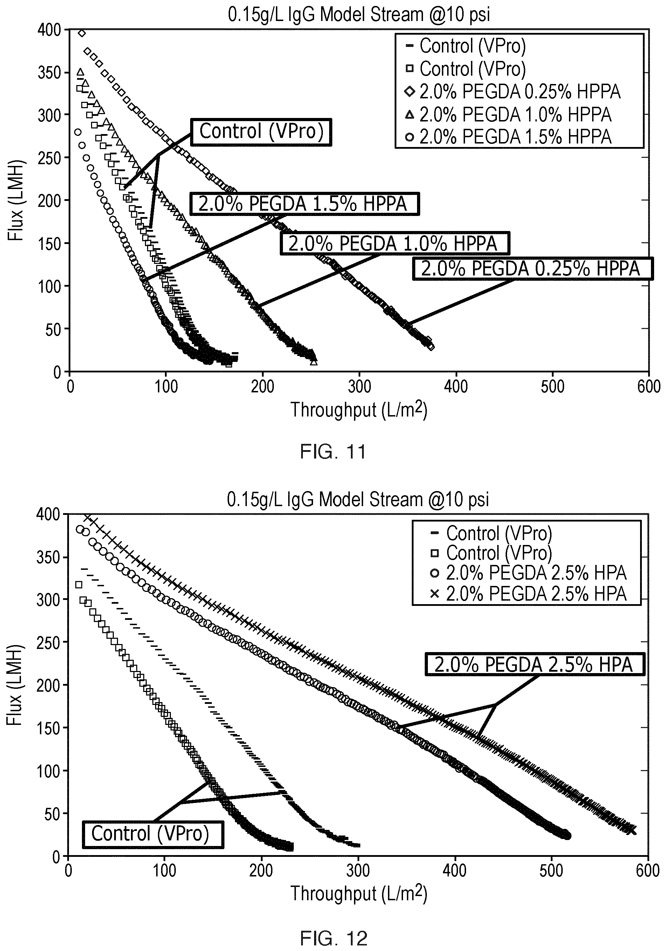

FIG. 11 depicts a graph plotting filtration flux of a single layer of surface treated membrane in LMH v. throughput in liters per squared-meters for a model stream operated at 10 psi, according to embodiments of the disclosure. As shown, membranes prepared with 2-Hydroxy-3-phenoxypropyl acrylate (HPPA) and Polyethylene glycol diacrylate (PEGDA) achieved higher throughput than the VPro Control.

### 2-Hydroxy-3-phenoxypropyl acrylate (HPPA), e.g., Cas#16969-10-1

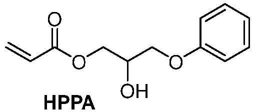

FIG. 12 depicts a graph plotting filtration flux of a single layer of surface treated membrane in LMH v. throughput in liters per squared-meters for a model stream operated at 10 psi, according to embodiments of the disclosure. As shown, membranes prepared with Hydroxypropyl acrylate (HPA) and Polyethylene glycol diacrylate (PEGDA) achieved higher throughput than the VPro Control.

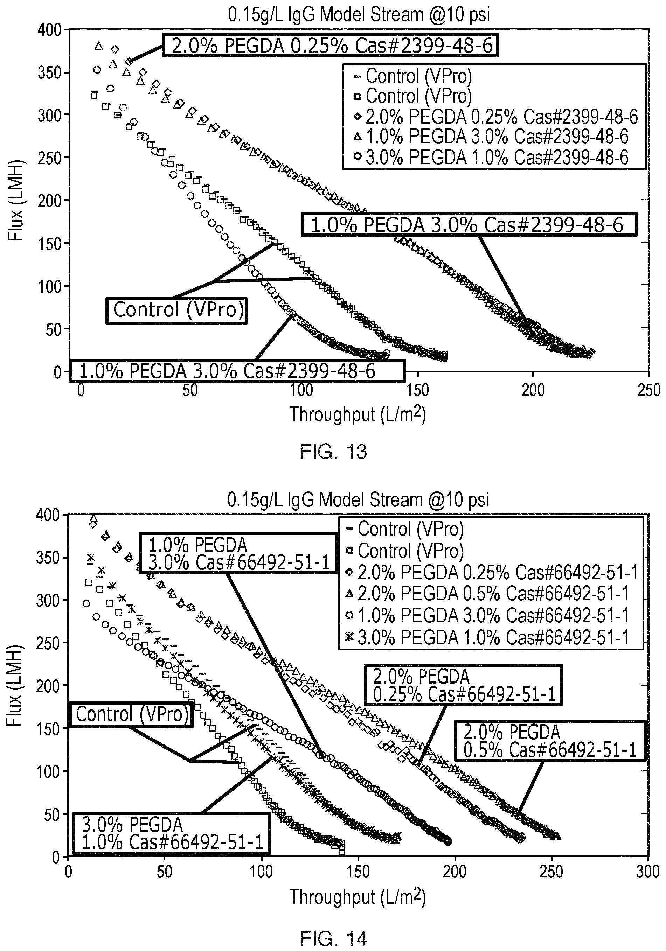

FIG. 13 depicts a graph plotting filtration flux of a single layer of surface treated membrane in LMH v. throughput in liters per squared-meters for a model stream operated at 10 psi, according to embodiments of the disclosure. As shown, membranes prepared with Tetrahydrofurfuryl acrylate (Cas#2399-48-6) and Polyethylene glycol diacrylate (PEGDA) achieved higher throughput than the VPro Control.

### Tetrahydrofurfuryl acrylate e.g., Cas#2399-48-6

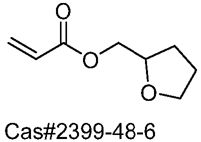

FIG. 14 depicts a graph plotting filtration flux of a single layer of surface treated membrane in LMH v. throughput in liters per squared-meters for a model stream operated at 10 psi, according to embodiments of the disclosure. As shown, membranes prepared with (5-Ethyl-1,3-dioxan-5-yl)methyl acrylate (Cas#66492-51-1) and Polyethylene glycol diacrylate (PEGDA) achieved higher throughput than the VPro Control.

### (5-Ethyl-1,3-dioxan-5-yl)methyl acrylate e.g., Cas#66492-51-1

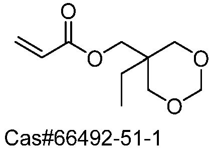

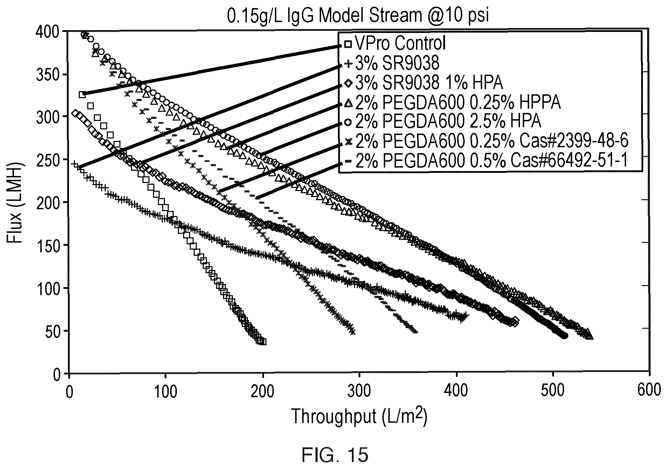

FIG. 15 depicts a graph plotting filtration flux of a single layer of surface treated membrane in LMH v. throughput in liters per squared-meters for a model stream operated at 10 psi, according to embodiments of the disclosure. As shown, membranes prepared with Bisphenol A ethoxylate diacrylate (Cas#64401-02-1) alone or with hydroxy propyl acrylate (HPA), Polyethylene glycol diacrylate (PEGDA) with one of 2-Hydroxy-3-phenoxypropyl acrylate (HPPA), hydroxy propyl acrylate (HPA), Tetrahydrofurfuryl acrylate (Cas#2399-48-6), or (5-Ethyl-1,3-dioxan-5-yl)methyl acrylate (Cas#66492-51-1) achieved higher throughput than the VPro Control.

Embodiments of the disclosure include a filtration device. In some embodiments, the filtration device is a polyethersulfone membrane, which is optionally an asymmetric porous membrane having a surface modification. In some embodiments, the surface modification is a cross-linked coating on the membrane, wherein the cross-linked coating is formed from a coating solution comprising a cross-linkable diacrylate.

In some embodiments, the coating solution used to make the cross-linked coating disposed on the membrane comprises at least one of 2.4-3.4% Hydroxypropyl acrylate and 1.1-2.1% polyethylene glycol diacrylate; 2.5-3.5% Bisphenol A ethoxylate diacrylate; 3.0-4.0% Bisphenol A ethoxylate diacrylate and 1.5-2.5% Hydroxypropyl acrylate; 1.5-2.5% Hydroxypropyl acrylate and 2.0-3.0% Polyethylene glycol diacrylate; 3.0-4.0% Bisphenol A ethoxylate diacrylate; 0.25-2.5% (2-Hydroxy-3-phenoxypropyl acrylate and 2.0-5.0% Polyethylene glycol diacrylate; 0.2-3.0% Tetrahydrofurfuryl acrylate and 1.0-2.5% Polyethylene glycol diacrylate; 0.2-3.0% (5-Ethyl-1,3-dioxan-5-yl)methyl acrylate and 1.0-2.5% Polyethylene glycol diacrylate; or 0.25-2.5% Propoxylated tetrahydrofurfuryl acrylate and 2.0-3.0% Polyethylene glycol diacrylate.

In some embodiments, the filtration device can be used to filter a biological fluid, wherein an aromatic excipient is used. In some embodiments, the aromatic excipient comprises at least one of caffeine, pyridoxine, phenylalanine, pyridoxal, pyridoxamine, theobromine, theophyline, xanthine, tyrosine, tryptophan, histidine, histamine, ascorbic acid, folic acid, or naphthalene-1-sulfonic acid.

It is to be understood that membranes having the cross-linked surface coating can be used in conjunction with the use of the excipients described herein. Furthermore, all filtration and/or purification methods of mAbs or fractionated plasma products described herein can use both the membranes having surface treatments and the excipients, separately or in conjunction therewith.

All ranges for formulations recited herein include ranges therebetween and can be inclusive or exclusive of the endpoints. Optional included ranges are from integer values therebetween (or inclusive of one original endpoint), at the order of magnitude recited or the next smaller order of magnitude. For example, if the lower range value is 0.2, optional included endpoints can be 0.3, 0.4, ... 1.1, 1.2, and the like, as well as 1, 2, 3 and the like; if the higher range is 8, optional included endpoints can be 7, 6, and the like, as well as 7.9, 7.8, and the like. One-sided boundaries, such as 3 or more, similarly include consistent boundaries (or ranges) starting at integer values at the recited order of magnitude or one lower. For example, 3 or more includes 4, or 3.1 or more.

Reference throughout this specification to "one embodiment," "certain embodiments," "one or more embodiments," "some embodiments," or "an embodiment" indicates that a feature, structure, material, or characteristic described in connection with the embodiment is included in at least one embodiment of the disclosure. Therefore, the appearances of the phrases such as "in one or more embodiments," "in certain embodiments," "in one embodiment," "some embodiments," or "in an embodiment" throughout this specification are not necessarily referring to the same embodiment.

Although some embodiments have been discussed above, other implementations and applications are also within the scope of the following claims. Although the specification describes, with reference to particular embodiments, it is to be understood that these embodiments are merely illustrative of the principles and applications of the present disclosure. It is therefore to be further understood that numerous modifications may be made to the illustrative embodiments and that other arrangements and patterns may be devised without departing from the spirit and scope of the embodiments according to the disclosure. Furthermore, particular features, structures, materials, or characteristics may be combined in any suitable manner in any one or more of the embodiments.

Publications of patent applications and patents and other non-patent references, cited in this specification are herein incorporated by reference in their entirety in the entire portion cited as if each individual publication or reference were specifically and individually indicated to be incorporated by reference herein as being fully set forth. Any patent application to which this application claims priority is also incorporated by reference herein in the manner described above for publications and references.
The present disclosure may be described by the following numbered paragraphs, denoted "para":
**1.** A filtration device for use with a biological fluid, comprising:
   a membrane; and
   a cross-linked coating on the membrane to form a surface modification,
   wherein the cross-linked coating is formed from a coating solution comprising a cross-linkable diacrylate.
**2.** The filtration device of para 1, wherein the coating solution used to make the cross-linked coating comprises:
   at least one of 2.4-3.4% Hydroxypropyl acrylate and 1.1-2.1% polyethylene glycol diacrylate; 2.5-3.5% Bisphenol A ethoxylate diacrylate; 3.0-4.0% Bisphenol A ethoxylate diacrylate and 1.5-2.5% Hydroxypropyl acrylate; 1.5-2.5% Hydroxypropyl acrylate, and 2.0-3.0% Polyethylene glycol diacrylate; 0.25-2.5% 2-Hydroxy-3-phenoxypropyl acrylate and 2.0-5.0% Polyethylene glycol diacrylate; or 3.0-4.0% Bisphenol A ethoxylate diacrylate.
**3.** The filtration device of para 1, wherein the membrane is a porous membrane.
**4.** The filtration device of para 1, wherein the membrane is a symmetric porous membrane or an asymmetric porous membrane.
**5.** The filtration device of para 1, wherein the membrane comprises a polyethersulfone material.
**6.** The filtration device of para 1, further comprising a vent.
**7.** The filtration device of para 1, wherein the filtration device is a virus filtration device.
**8.** A method for making a membrane for filtering biological fluids, comprising:
   applying a cross-linkable diacrylate solution to a porous membrane; and
   initiating a curing of the cross-linkable solution to form a cured coating on the porous membrane, wherein the cured coating promotes a high flux filtration device.
**9.** The method of para 8, wherein the cross-linkable diacrylate solution used to make the cross-linked coating comprises a solution of at least one of 2.4-3.4% Hydroxypropyl acrylate and 1.1-2.1% polyethylene glycol diacrylate; 2.5-3.5% Bisphenol A ethoxylate diacrylate; 3.0-4.0% Bisphenol A ethoxylate diacrylate and 1.5-2.5% Hydroxypropyl acrylate; 1.5-2.5% Hydroxypropyl acrylate and 2.0-3.0% Polyethylene glycol diacrylate; 0.25-2.5% 2-Hydroxy-3-phenoxypropyl acrylate and 2.0-5.0% Polyethylene glycol diacrylate; or 3.0-4.0% Bisphenol A ethoxylate diacrylate.
**10.** The method of para 8, wherein the coating solution is applied to the membrane by a dipping or a spraying step.
**11.** The method of para 8, wherein the method for initiating cross-linking reaction is the application of E-beam, ultraviolet light, or high-energy radiation.
**12.** The method of para 8, wherein the membrane is housed in a housing further comprising a biological fluid input and a biological fluid output.
**13.** A method for filtering a biological fluid, comprising:
   housing a surface treated porous membrane in a filtration device having an input and an output, wherein the surface treatment comprises a cross-linked diacrylate coating;
   introducing a biological fluid into the input of the filtration device; and
   collecting a filtrate from the output.
**14.** The method of para 13, wherein the membrane comprises a polyethersulfone material.
**15.** The method of para 13, wherein the filtration device is a dead-end filter or a tangential-flow filter.
**16.** The method of para 13, wherein the biological fluid comprises a monoclonal antibody solution, a protein-containing solution, a solution containing fragment antigen-binding fragments, a solution containing recombinant proteins, or a fractionated plasma product.
**17.** A surface treatment for a membrane, comprising:
   a cross-linkable diacrylate solution used to make the cross-linked coating, wherein the cross-linked coating comprises a solution of at least one of 2.4-3.4% Hydroxypropyl acrylate; and 1.1-2.1% polyethylene glycol diacrylate; 2.5-3.5% Bisphenol A ethoxylate diacrylate; 3.0-4.0% Bisphenol A ethoxylate diacrylate and 1.5-2.5% Hydroxypropyl acrylate; 1.5-2.5% Hydroxypropyl acrylate and 2.0-3.0% Polyethylene glycol diacrylate; 0.25-2.5% 2-Hydroxy-3-phenoxypropyl acrylate and 2.0-5.0% Polyethylene glycol diacrylate; or 3.0-4.0% Bisphenol A ethoxylate diacrylate.
**18.** A filtration device for use with a biological fluid, comprising:
   a membrane; and
   a cross-linked coating disposed on the membrane to form a cross-linked, copolymeric surface modification or treatment, wherein the cross-linked coating is formed from a coating solution comprising a cross-linkable diacrylate, wherein the coating solution is 2.4-3.4% Hydroxypropyl acrylate and 1.1-2.1% polyethylene glycol diacrylate or 3.0-4.0% Bisphenol A ethoxylate diacrylate and 1.5-2.5% Hydroxypropyl acrylate; or 1.5-2.5% Hydroxypropyl acrylate and 2.0-3.0% Polyethylene glycol diacrylate, or 0.25-2.5% 2-Hydroxy-3-phenoxypropyl acrylate and 2.0-5.0% Polyethylene glycol diacrylate.
**19.** The filtration device of para 18, further comprising a vent.
**20.** The filtration device of para 18, wherein the filtration device is a virus filtration device.

## Claims

1. A filtration device for use with a biological fluid, comprising:
a membrane; and
a cross-linked coating on the membrane to form a surface modification,
wherein the cross-linked coating is formed from a coating solution comprising a cross-linkable diacrylate;
wherein the coating solution used to make the cross-linked coating comprises at least one of:
3.0-4.0% Bisphenol A ethoxylate diacrylate; and
2.5-3.5% Bisphenol A ethoxylate diacrylate.

2. The filtration device of claim 1, wherein the membrane is a porous membrane.

3. The filtration device of claim 1, wherein the membrane is a symmetric porous membrane or an asymmetric porous membrane.

4. The filtration device of claim 1, wherein the membrane comprises a polyethersulfone material.

5. The filtration device of claim 1, further comprising a vent.

6. The filtration device of claim 1, wherein the filtration device is a virus filtration device.

7. A method for filtering a biological fluid, comprising:
housing a surface treated porous membrane in a filtration device having an input and an output, wherein the surface treatment comprises a cross-linked diacrylate coating,
introducing a biological fluid into the input of the filtration device; and
collecting a filtrate from the output;
wherein the cross-linked diacrylate coating comprises a solution of at least one of:
3.0-4.0% Bisphenol A ethoxylate diacrylate; and
2.5-3.5% Bisphenol A ethoxylate diacrylate.

8. The method of claim 7, wherein the membrane comprises a polyethersulfone material.

9. The method of claim 7, wherein the filtration device is a dead-end filter or a tangential-flow filter.

10. The method of claim 7, wherein the biological fluid comprises a monoclonal antibody solution, a protein-containing solution, a solution containing fragment antigen-binding fragments, a solution containing recombinant proteins, or a fractionated plasma product.

11. A surface treatment for a membrane, comprising:
a cross-linkable diacrylate solution used to make the cross-linked coating,
wherein the cross-linked coating comprises a solution of at least one of:
3.0-4.0% Bisphenol A ethoxylate diacrylate; and
2.5-3.5% Bisphenol A ethoxylate diacrylate.

12. The method of claim 7, further comprising a vent in the filtration device.

13. The method of claim 7, wherein the filtration device is a virus filtration device.
